# EUROPÄISCHE PATENTSCHRIFT

(11) **EP 3 320 991 B1**
(45) Veröffentlichungstag und Bekanntmachung des Hinweises auf die Patenterteilung: **31.10.2018**
(21) Anmeldenummer: 16198281.4
(22) Anmeldetag: 10.11.2016
(51) Int. Cl.: B09B 3/00, B02C 19/00, B09B 5/00, A61B 50/36, A61B 50/30

(54) **VERFAHREN ZUR BEHANDLUNG VON METALLISCHEN MEDIZINISCHEN GERÄTEN SOWIE METALLISCHE MEDIZINISCHE GERÄTE ODER METALLISCHE GEBINDE ERHALTEN NACH DIESEM VERFAHREN**
METHOD FOR TREATING METALLIC MEDICAL DEVICES AND METALLIC MEDICAL DEVICES OR METALLIC CONTAINER OBTAINED BY THIS METHOD
PROCÉDÉ DE TRAITEMENT D'APPAREILS MÉDICAUX MÉTALLIQUES AINSI QU'APPAREILS MÉDICAUX MÉTALLIQUES OU EMBALLAGES MÉTALLIQUES OBTENUS SELON CE PROCÉDÉ

(43) Veröffentlichungstag der Anmeldung: 16.05.2018
(73) Patentinhaber: Lorke, Werner W., 60486 Frankfurt/M. (DE)
(72) Erfinder: Lorke, Werner W., 60486 Frankfurt/M. (DE)
(74) Vertreter: Metten, Karl-Heinz

(56) Entgegenhaltungen:
- EP-A2- 0 350 406
- FR-A1- 2 842 111
- JP-A- H0 449 971
- JP-A- H02 160 089
- US-A- 3 476 506
- US-A1- 2002 100 706
- US-A1- 2016 015 388

## Beschreibung

Die vorliegende Erfindung betrifft ein Verfahren zur, insbesondere Ressourcen schonenden bzw. Wertstoff erhaltenden, Behandlung, insbesondere zur recycelnden Entsorgung, von, insbesondere nicht-infektiös kontaminierten, metallischen medizinischen Geräten aus insbesondere Kliniken, medizinischen Ambulanzen und/oder Arztpraxen. Ferner betrifft die Erfindung metallische medizinische Geräte oder metallische Gebinde erhalten oder erhältlich gemäß dem erfindungsgemäßen Verfahren.

Metallische Klinikabfälle wie medizinische Implantate, Endoprothesen und chirurgischen Ein- oder Mehrweg-Instrumente werden im Allgemeinen je nach Kontaminationsgrad einer hierfür vorgesehenen Endlagerstätte zugeführt. Nicht selten werden solche metallischen Klinikabfälle zunächst zusammen mit weiteren Klinikabfällen einer Müllverbrennung unterzogen, und die hierbei erhaltenen Rückstände werden sodann deponiert. Auch geht man gegenwärtig davon aus, dass pro Jahr in Deutschland etwa 50.000 kg medizinische Implantate und defekte chirurgische Mehrweg-Instrumente im Müll landen. Darüber hinaus nimmt der Verbrauch von chirurgischen Einweginstrumenten - vorwiegend aus chromhaltigen Edelstählen gefertigt - insbesondere in Kliniken, Ambulanzen und Arztpraxen seit Jahren zu. Die Menge an Edelstahl, die allein in Deutschland in 2014 für chirurgische Einweginstrumente verwendet wurde, lag bei etwa 8000 t. Auch dieser Edelstahlabfall wird regelmäßig über den allgemeinen Klinikabfall in Müllverbrennungsanlagen entsorgt. Die metallischen Klinikabfälle werden folglich dem Wertstoffkreislauf dauerhaft entzogen.

Die EP 350 406 A2 betrifft ein Verfahren zum Entsorgen von medizinischem Abfallmaterial, umfassend das Ablegen medizinischer Abfallmaterialien in einen Behälter, Versiegeln des Behälters, Platzieren des versiegelten Behälters zusammen mit weiterem Metallmaterial in einem Metallschmelzofen und Erhitzen des Ofens auf eine Temperatur, die ausreicht, um den Behälter und die medizinischen Abfallmaterialien vollständig zu verbrennen oder zu schmelzen. Ferner betrifft die EP 350 406 A2 eine Vorrichtung zum Entsorgen von medizinischen Abfällen, umfassend einen Behälter mit einer Öffnung, einen Deckel, der die Öffnung des Behälters verschließen kann, eine Basis, auf der der Behälter montiert werden kann, einen vertikalen Rahmen, der sich von der Basis nach oben erstreckt, eine Abdeckung, die schwenkbar an dem Rahmen angebracht ist und zwischen einer offenen Position, in der Abfallmaterialien in die Öffnung des Behälters eingebracht werden können, und einer geschlossenen Position, in der die Öffnung des Behälters durch den Deckel verschlossen ist, bewegbar ist, ein Fußpedal und ein Mittel zum Öffnen der Abdeckung, wenn eine Person auf das Fußpedal tritt. Mit dem in der EP 350 406 A2 offenbarten Verfahren sowie mit der darin offenbarten Vorrichtung sollen sich medizinische Abfallmaterialien sicher und zuverlässig aus Krankenhäusern entsorgen lassen, ohne dass diese zuvor vom Personal sortiert werden müssen. Auf diese Weise soll sich auch die Gefahr von Sekundärinfektionen verringern lassen.

Der vorliegenden Erfindung lag die Aufgabe zugrunde, die aus dem Stand der Technik bekannten Nachteile abzustellen oder zu mindern. Insbesondere lag der Erfindung die Aufgabe zugrunde, die Handhabung von metallischen Klinikabfällen zu verbessern.

Demgemäß wurde ein Verfahren zur, insbesondere Ressourcen schonenden, Behandlung, insbesondere zur recycelnden Entsorgung, von, insbesondere kontaminierten, besonders bevorzugt nicht-infektiös kontaminierten, metallischen medizinischen Geräten gefunden, umfassend die Schritte:
a) Zurverfügungstellung mindestens eines mindestens abschnittsweise flexiblen, verschließbaren Sammelbehältnisses für benutzte und/oder kontaminierte metallische medizinische Geräte und mindestens eines verschließbaren Metallgebindes, eingerichtet und ausgelegt zur Aufnahme von mindestens zwei, insbesondere einer Vielzahl an verschlossenen mindestens abschnittsweise flexiblen Sammelbehältnissen, enthaltend benutzte und/oder kontaminierte metallische medizinische Geräte,
b) Sammeln von benutzten und/oder kontaminierten metallischen medizinischen Geräten in dem mindestens einen mindestens abschnittsweise flexiblen, verschließbaren Sammelbehältnis,
c) gegebenenfalls Verschließen, insbesondere irreversibles Verschließen, des mit benutzten und/oder kontaminierten metallischen medizinischen Geräten befüllten mindestens einen mindestens abschnittsweise flexiblen Sammelbehältnisses,
d) Überführen des mindestens einen, insbesondere verschlossenen, mindestens abschnittsweise flexiblen Sammelbehältnisses in das verschließbare, insbesondere starre, Metallgebinde,
e) Verschließen des mit dem mindestens einen, insbesondere verschlossenen, mindestens abschnittsweise flexiblen Sammelbehältnis befüllten Metallgebindes,
f) Unterwerfen des mindestens einen verschlossenen Metallgebindes mindestens einem Pressschritt zur Volumenreduktion unter Erhalt eines verpressten Gebildes und
g) Aufschmelzen des Metalls des verpressten Gebildes enthaltend das Metall des Metallgebindes und das Metall der metallischen medizinischen Geräte und Rückgewinnung der aufgeschmolzenen Metalle.

In einer vorteilhaften Ausgestaltung des erfindungsgemäßen Verfahrens umfasst dieses ferner den Schritt die Herstellung von metallischen medizinischen Geräten und/oder von metallischen Gebinden aus den in Schritt g) rückgewonnenen Metallen (Schritt h)). Dieser Schritt kann dabei beispielsweise auch die Bereitstellung von aus in Schritt g) rückgewonnenen Metallen bzw. Metalllegierungen, und damit von in ihrer chemischen Zusammensetzung bekannten Metall- bzw. Legierungszuschlägen für u.a. die Herstellung von metallischen medizinischen Geräten und/oder von metallischen Gebinden umfassen.

Das Sammelbehältnis ist in einer bevorzugten Ausgestaltung nicht nur abschnittsweise, sondern im Wesentlichen vollständig aus flexiblen, beispielsweise knick-, dehn- und/oder faltbaren, Wandungsmaterialien gebildet bzw. im Wesentlichen als Ganzes flexibel ausgestaltet.

Metallische medizinische Geräte, die mit dem erfindungsgemäßen Verfahren grundsätzlich einem Recyclingprozess zugeführt werden können, umfassen z.B. gebrauchte und/oder nicht verwendete Endoprothesen, chirurgische Instrumente, insbesondere Mehrweginstrumente, medizinische Einweginstrumente, Herzschrittmacher und/oder orthopädische Zubehörteile. Bei den nach dem erfindungsgemäßen Verfahren behandelten metallischen medizinischen Geräten handelt es sich vorzugsweise um metallische Einweg-Instrumente und/oder um metallische Mehrweg-Instrumente, insbesondere chirurgische Mehrweg-Instrumente. Mit dem erfindungsgemäßen Verfahren können insbesondere auch solche metallischen medizinischen Geräte einer Wiederverwertung zugeführt werden, die Titan, Kobalt, Chrom und/oder Nickel enthalten. Derartige metallische medizinische Geräte sind dabei häufig aus mit Titan, Kobalt, Chrom und/oder Nickel legierten Edelstählen gebildet. Mit dem erfindungsgemäßen Verfahren lassen sich ganz besonders bevorzugt derartige metallischen medizinische Geräte, insbesondere metallische Einweg-Instrumente und metallische chirurgische Mehrweg-Instrumente, einer Wiederverwertung zuführen, die Chrom und/oder Nickel legierte Edelstähle enthalten.

Gemäß dem erfindungsgemäßen Verfahren sind solche flexiblen, verschließbaren Sammelbehältnisse besonders geeignet, die im Wesentlichen feuchtigkeitsbeständig, dicht, insbesondere flüssigkeitsdicht, perforationsresistent, insbesondere stichfest, reißfest und/oder dauerhaft verschließbar sind. Die flexiblen, verschließbaren Sammelbehältnisse sind zweckmäßiger Weise eingerichtet und ausgelegt, um benutzte und/oder kontaminierte metallische medizinische Geräte mit einem Gesamtgewicht im Bereich von 0,5 bis 5,0 kg. insbesondere im Bereich von 1,0 bis 3,0 kg, aufzunehmen.

In einer vorteilhaften Ausgestaltung umfassen geeignete Sammelbehältnisse eine flexible Behältnisseitenwandung, Behältnisboden und einen dem Behältnisboden gegenüberliegenden verschlossenen Behältnisrand, wobei eine Behältnisöffnung näher beabstandet zum verschlossenen Behältnisrand als zum Behältnisboden in einem Abschnitt der Behältnisseitenwandung vorliegt. Dabei sind auch solche flexiblen Sammelbehältnisse geeignet, die eine eine flexible Behältnisseitenwandung, einen Behältnisboden, einen dem Behältnisboden gegenüberliegenden Behältnisrand und eine Behältnisöffnung enthalten und bei denen die Behältnisseitenwandung, insbesondere in Form von Vorderwand, Rückwand und gegebenenfalls Seitenwand oder -wänden. und insbesondere auch einschließlich Behältnisboden und/oder Behältnisrand, aus mindestens einer Polyolefin-Faservlieslage, einem Laminatsystem, enthaltend mindestens eine Papierlage und mindestens eine Kunststofffolienlage, und/oder mindestens eine feuchtigkeitsbeständig beschichtetet weitere Papierlage und/oder einer Lage aus Polyvinylbutyrat, insbesondere recyceltem Polyvinylbutyrat, gebildet ist. Darüber hinaus kann auch auf solche flexiblen Sammelbehältnisse zurückgegriffen werden, enthaltend eine Vorderseitenwandung und eine Rückseitenwandung und gegebenenfalls gegenüberliegende Seitenwandungen, insbesondere Seitenfaltenwandungen, einen den oberen, verschlossenen Behältnisrand bildenden Falz, über den Vorderseitenwandung und Rückseitenwandung miteinander verbunden sind, und/oder einen den oberen, verschlossenen Behältnisrand bildenden Behältnisabschnitt, über den Vorderseitenwandung und Rückseitenwandung miteinander in Verbindung stehen und/oder ineinander übergehen, ferner eine Öffnung, insbesondere schlitzförmige Öffnung, im oberen Bereich der Vorderseitenwandung sowie einen Behälterboden, der mindestens zwei, insbesondere eine Vielzahl an leporelloartig gefalteten Segmenten aufweist, wobei dieser Behälterboden vorzugsweise einstückig mit der Vorderseitenwandung oder der Rückseitenwandung vorliegt und/oder das endständige Segment der leporelloartig gefalteten Segmente des Behälterbodens mit der Innenwandung der Vorderseitenwandung oder der Rückseitenwandung verbunden, insbesondere verklebt, ist.

Bei den geeigneten flexiblen Sammelbehältnissen liegt vorzugsweise mindestens einen Klebestreifen in dem Abschnitt des Vorderseitenareals zwischen der Beutelöffnung und der Falz oder dem Behältnisabschnitt auf derjenigen Seite des Vorderseitenareals vor, die bei einem aus der Materialbahn gebildeten Sammelbehältnis die Außenseite bildet.

Als besonders geeignet hat sich ein Sammelbehältnis für metallische medizinische Geräte, insbesondere für metallische chirurgische Einweginstrumente, erwiesen, umfassend eine, insbesondere flexible, Behältnisvorderwand, eine, insbesondere flexible, Behältnisrückwand, gegebenenfalls eine erste, insbesondere flexible, Behältnisseitenwand, umfassend mindestens eine Seitenfalte, gegebenenfalls eine der ersten Behältnisseitenwand gegenüberliegende zweite, insbesondere flexible, Behältnisseitenwand, umfassend mindestens eine Seitenfalte, ein unteres Behältnisende, umfassend einen Behältnisboden, und einen dem Behältnisboden gegenüberliegendes verschlossenes oberes Behältnisende, wobei näher beabstandet zum verschlossenen oberen Behältnisende als zum Behältnisboden in einem Abschnitt der Behältnisvorderwand oder der Behältnisrückwand eine Behältnisöffnung vorliegt, die sich in Richtung von der ersten Behältnisseitenwand zu der zweiten Behältnisseitenwand erstreckt, ferner umfassend mindestens ein Verschlusselement an oder auf der Behältniswand, welche die Behältnisöffnung enthält, diesseits der Behältnisöffnung in Bezug auf das obere Behältnisende, ausgelegt und eingerichtet, um die Behältnisöffnung, insbesondere irreversibel, zu verschließen, und mindestens eine Haltevorrichtung, insbesondere Griff oder Schlaufe, ausgelegt und eingerichtet zum temporären Befestigen des Sammelbehältnisses, so dass bei gattungsgemäßem Gebrauch die Behältnisöffnung unterhalb des oberen Behältnisende vorliegt, wobei Behältnisvorderwand und Behältnisrückwand am unteren Behältnisende, insbesondere über eine Faltung, einstückig ineinander übergehen oder wobei Behältnisvorderwand und Behältnisrückwand am unteren Behältnisende in einem sich von der ersten Behältnisseitenwand bis zur zweiten Behältnisseitenwand erstreckenden überlappenden ersten Abschnitt, insbesondere irreversibel, miteinander verbunden vorliegen und wobei der überlappende verbundene erste Abschnitt vollständig oder teilweise umgeschlagen auf und, insbesondere irreversibel, verbunden mit der Behältnisvorderwand oder die Behältnisrückwand vorliegt und wobei Behältnisvorderwand und Behältnisrückwand am verschlossenen oberen Behältnisende in einem sich von der ersten Behältnisseitenwand bis zur zweiten Behältnisseitenwand erstreckenden überlappenden zweiten Abschnitt zumindest teilweise, insbesondere irreversibel, miteinander verbunden vorliegen und wobei der überlappende zumindest teilweise verbundene zweite Abschnitt vollständig oder teilweise umgeschlagen auf und, insbesondere irreversibel, verbunden mit der Behältnisvorderwand oder die Behältnisrückwand vorliegt oder wobei ein am oberen Behältnisende über die Behältnisrückwand hinausragender Abschnitt der Behältnisvorderwand unter Ausbildung des verschlossenen oberen Endes umgeschlagen auf und, insbesondere irreversibel, verbunden mit der Behältnisrückwand vorliegt oder wobei ein am oberen Behältnisende über die Behältnisvorderwand hinausragender Abschnitt der Behältnisrückwand unter Ausbildung des verschlossenen oberen Endes umgeschlagen auf und, insbesondere irreversibel, verbunden mit der Behältnisvorderwand vorliegt. Auch mit diesem Sammelbehältnis lassen sich metallische medizinische Geräte in Form von Einweg- und/oder Mehrweg-Instrumenten, insbesondere Einweg-Instrumenten, sammeln und (zwischen) lagern. In den Sammelbehältnissen können z.B. metallische Kanülen, metallische Skalpelle, metallische Pinzetten, metallische Scheren, mechanische metallische Klemmen und/oder metallische Zangen gesammelt werden.

Im Zusammenhang mit den vorangehend geschilderten Ausführungsformen geeigneter Sammelbehältnisse soll im Sinne der Erfindung unter irreversibel verschlossen bzw. irreversibel verbunden in einer besonders zweckmäßigen Ausgestaltung verstanden werden, dass ein Lösen der irreversibel verschlossenen oder irreversibel verbundenen Gegenstände nicht zerstörungsfrei möglich ist.

Geeignete Sammelbehältnisse sind vorzugsweise im Wesentlichen vollständig aus einem flexiblen Material gefertigt.

Bei den geeigneten Sammelbehältnissen ist die Behältnisöffnung vorzugsweise in der Form eines Schlitzes ausgebildet ist, welcher zweckmäßigerweise im Wesentlichen parallel zum Behältnisboden und/oder zum verschlossenen oberen Behältnisende, insbesondere Behältnisrand, verläuft. Die Behältnisöffnung, insbesondere der Schlitz, kann dabei eine Ausstanzung oder einen Schnitt in der Behältniswandung darstellen. Vorzugsweise ist die Behältnisöffnung ausgelegt und eingerichtet, um metallische Einweg- und/oder Mehrweg-Instrumente, insbesondere metallische chirurgische Einweginstrumente, aufzunehmen und/oder um in dem Sammelbehältnis vorliegende metallische Einweg- und/oder Mehrweginstrumente, insbesondere metallische chirurgische Einweginstrumente, nicht durch Umstülpen wieder freizugeben. Vielfach hat es sich als vorteilhaft erwiesen, die Behältnisöffnung mit einem, insbesondere umlaufend, verstärkten Behältnisrand auszustatten.

Der Behältnisboden am unteren Behältnisende kann z.B. durch eine Faltung bzw. Bodenfalz gebildet werden. Alternativ kann der Behältnisboden durch eine Bodenwand darstellen. Bevorzugt ist der Behältnisboden auch verstärkt ausgebildet, beispielsweise durch einen doppel- oder mehrlagigen Abschnitt.

Die Haltevorrichtung kann in einer geeigneten Ausgestaltung auch ein Loch in dem Sammelbehältnis, insbesondere der Behältnisvorderwand und/oder der Behältnisrückwand, beispielsweise Griffloch, darstellen. Das Loch kann in einer Ausführungsform auch ausgelegt und eingerichtet sein, um eine Halteschlaufe oder -kordel hindurchzufädeln, welche dann zum temporären Befestigen des Sammelbehältnisses genutzt werden kann. Vorzugsweise wird das Loch in dem Sammelbehältnis derart ausgestaltet, dass ein Zugang zu dem Innenraum des Sammelbehältnisses über dieses Loch nicht möglich ist. Beispielsweise können die Lochränder von im Wesentlichen deckungsgleichen Löchern in Behältnisvorderwand und Behältnisrückwand umlaufend verschweißt oder verklebt sein.

Das Verbinden von Behältniswandungsabschnitten kann mittels Verklebens und/oder, insbesondere thermischen, Verschweißens und/oder Vernietens oder ähnlicher mechanischer Verbindungsmechanismen erfolgen.

Besonders bevorzugt sind geeignete Sammelbehältnisse im Wesentlichen einstückig ausgebildet. Hierbei wird das Verschlusselement in der Regel außer Betracht gelassen. Das Verschlusselement für die Behältnisöffnung stellt vorzugsweise mindestens einen Klebestreifen dar.

Für die flexiblen Wandungsmaterialien des flexiblen Sammelbehältnisses kann insbesondere auch auf Polyolefin-Faservlieslagen, Laminatsystemen, enthaltend mindestens eine Papierlage und/oder mindestens eine feuchtigkeitsbeständig beschichtete weitere Papierlage und/oder mindestens eine Kunststofffolienlage, insbesondere enthaltend mindestens eine Papierlage und mindestens eine Kunststofffolienlage und/oder mindestens eine feuchtigkeitsbeständig beschichtetete weitere Papierlage, ein- oder beidseitig in eine Kunststofflage eingebettete Metallgewebe, Metallgewirke oder Metallvliese, oder auf Lagen aus Polyvinylbutyrat, insbesondere recyceltem Polyvinylbutyrat, zurückgegriffen werden. Die flexiblen Sammelbehältnisse werden nach Befüllen mit benutzten und/oder kontaminierten metallischen Geräten aus Kliniken oder Arztpraxen verschlossen. Die flexiblen, verschließbaren Sammelbehältnisse können demgemäß für benutzte und/oder kontaminierte metallische medizinische Geräte, die in Kliniken anfallen, ebenso benutzt werden wie für entsprechend benutzte und/oder kontaminierte metallische medizinische Geräte aus Arztpraxen.

Insbesondere für den Einsatz im Klinikbetrieb hat es sich als vorteilhaft erwiesen, wenn die flexiblen, verschließbaren Sammelbehältnisse eingerichtet und ausgelegt sind, um an einem Stationswagen temporär aufgehängt zu werden. Dies kann z.B. über integral in dem Sammelbehältnis vorliegende oder über extern angebrachte Halteschlaufen bewerkstelligt werden.

Vielfach hat es sich als besonders vorteilhaft erwiesen, die Öffnung der Sammelbehältnisse in der Form eines Schlitzes auszuführen, der in der Vorder- oder der Rückseitenwandung unterhalb des oberen Randes des Sammelbehältnisses vorliegt. Eine derartige Öffnung lässt sich durch einen Klebestreifen mit einem geeigneten Klebermaterial, das dem Fachmann bekannt ist, in einer Weise verschließen, dass ein Abtrennen des auf den die Öffnung aufgebrachten Klebestreifens nicht mehr oder nicht mehr zerstörungsfrei möglich ist. Eine schlitzförmige Öffnung im Sinne der vorliegenden Erfindung umfasst sowohl einen Schlitz als solchen, beispielsweise herbeigeführt durch einen Schnitt, wie auch eine Öffnung, die sich über die Breite des Sammelbehältnissen erstreckt und die gleichzeitig auch eine, insbesondere geringfügige, Erstreckung in Richtung von dem oberen Ende zu dem unteren Ende des Sammelbehältnisses aufweist. Derartige Öffnungen können beispielsweise mittels Ausstanzung erzeugt werden. Bevorzugt sind regelmäßig solche schlitzförmigen Öffnungen, die im Wesentlichen nicht über eine Erstreckung in Richtung von dem oberen Ende zu dem unteren Ende verfügen. Die sich gegenüberliegenden seitlichen Endpunkte der schlitzförmigen Öffnung können zum Beispiel in rundliche Ausnehmungen oder Ausstanzung münden. Hierdurch kann ein unbeabsichtigtes Einreißen der schlitzförmigen Öffnung unterbunden bzw. entgegengewirkt werden.

In einer bevorzugten Ausführungsform wird das flexible Sammelbehältnis nach der, insbesondere vollständigen, Befüllung und vorzugsweise spätestens vor der Überführung in das Metallgebinde verschlossen, vorzugsweise irreversibel verschlossen.

Mit dem erfindungsgemäßen Verfahren ist es auch möglich, flexible Sammelbehältnisse zu verwenden, die, insbesondere ausschließlich, nicht-infektiös kontaminierte metallische Abfälle enthalten. Bevorzugt sind hierbei flexible Sammelbehältnisse, die, insbesondere ausschließlich, nicht-infektiös kontaminierte metallische Abfälle gemäß Abfallschlüssel AS 180104 nach Merkblatt Nr. 18 der Richtlinie über die ordnungsgemäße Entsorgung von Abfällen aus Einrichtungen des Gesundheitsdienstes gemäß Bundesgesundhbl. 2002, 51, 234 - 241, der Länder-Arbeitsgemeinschaft Abfall (LAGA) enthalten.

Vielfach hat es sich als zweckmäßig erwiesen, benutzte und/oder kontaminierte Kanülen und/oder Skalpelle nicht mit den vorangehend geschilderten flexiblen Sammelbehältnissen zu sammeln.

Die derart verschlossenen flexiblen Sammelbehältnisse werden gemäß dem erfindungsgemäßen Verfahren in ein Metallgebinde überführt. Dieses Metallgebinde verfügt regelmäßig über hartschalige Außenwände, die sich nicht ohne beträchtliche Krafteinwirkung verformen lassen. Dieses Metallgebinde ist vorzugsweise aus einem oder mehreren Stahlblechen gebildet und stellt beispielsweise ein Fass oder eine Tonne aus Stahl dar. Zweckmäßigerweise wird dieses Metallgebinde mithilfe einer Verschlussvorrichtung, beispielsweise mit einem Verschlussdeckel, verschlossen, der selber gegen mechanische Einflüsse stabil ist, beispielsweise ebenfalls aus Stahlblechen gefertigt ist. Die verschließbaren Metallgebinde verfügen zweckmäßiger Weise über ein Aufnahmevolumen im Bereich von 50 bis 250 1, insbesondere im Bereich von 100 bis 200 1.

Es hat sich in einer Ausführungsform des erfindungsgemäßen Verfahrens als zweckmäßig erwiesen, dass das Verschließen des Metallgebindes in Schritt e) den Schritt des Verplombens umfasst.

Eine wesentliche Behandlungsstufe des erfindungsgemäßen Verfahrens besteht darin, dass in Schritt f) jeweils ein einzelnes verschlossenes Metallgebinde, enthaltend die mit benutzten und/oder kontaminierten metallischen medizinischen Geräten befüllten flexiblen Sammelbehältnisse, oder eine Vielzahl an solchen verschlossenen Metallgebinden einem Pressschritt, unter Ausbildung eines verpressten, insbesondere im Wesentlichen zylinderförmigen, Gebildes unterworfen wird. Für diesen Pressschritt kann auf herkömmliche Metallpressen zurückgegriffen werden. Die mit den gefüllten flexiblen Sammelbehältnissen bestückten verschlossenen Metallgebinde werden in diesem Pressschritt im Allgemeinen in einer Weise zusammengepresst, dass allenfalls geringfügige Hohlräume in dem verpressten Gebilde verbleiben. Des Weiteren wird der Schritt des Verpressens vorzugsweise in einer Weise vorgenommen, dass das befüllte Metallgebinde hierbei nicht aufreißt oder aufplatzt.

Demgemäß sieht das erfindungsgemäße Verfahren vor, eine Vielzahl verschlossener, befüllter Metallgebinde gemäß Schritt e) zu einem ersten Lagerort, insbesondere umfassend eine Metallpresse, zu transportieren.

Insbesondere auch das verpresste Gebilde kann als solches gelagert werden, beispielsweise um abzuwarten, bis eine hinreichend große Anzahl an verpressten Gebilden vorliegt, die dem Folgeschritt der Metallschmelze zugeführt werden können.

Des Weiteren kann das erfindungsgemäßen Verfahren demgemäß in der Weise ausgestaltet werden, dass eine Vielzahl verpresster Gebilde gemäß Schritt f) zu einem zweiten Lagerort, insbesondere umfassend eine Metallschmelze, transportiert wird.

Die verpressten Gebilde können als solche dem Schmelzverfahren zugeführt werden. Hierbei hat sich als sehr vorteilhaft erwiesen, dass die benutzten und/oder kontaminierten metallischen medizinischen Geräte zunächst in dem flexiblen Sammelbehältnis gesammelt und aufbewahrt werden. Diese flexiblen Sammelbehältnisse, insbesondere wenn unter Verwendung der vorangehend spezifizierten Beutelmaterialien hergestellt, fallen in einer Menge an, dass die metallurgische Schmelze-und die Rückgewinnung der Metalle hierdurch nicht beeinflusst oder beeinträchtigt werden. Das flexible Sammelbehältnismaterial wird in der Regel im metallurgischen Aufbereitungsprozess, d.h. dem Aufschmelzprozess der metallischen medizinischen Geräte, ohne toxische Rückstände zu bilden, verbrannt. Hierdurch können die Vorgaben gemäß LAGA M 18 - Vollzugshilfe zur Entsorgung von Abfällen aus Einrichtungen des Gesundheitsdienstes (Mitteilung der Bund/Länder-Arbeitsgemeinschaft Abfall; Stand Januar 2015; LAGA - Bund/Länder-Arbeitsgemeinschaft Abfall) und auch die Technischen Regeln für Biologische Arbeitsstoffe TRBA 250 betreffend Biologische Arbeitsstoffe im Gesundheitswesen und in der Wohlfahrtspflege erfüllt werden, welche das Entleeren und Sortieren von kontaminierten Klinikabfällen verbieten.

Mit der vorliegenden Erfindung geht die überraschende Erkenntnis einher, dass metallische medizinische Geräte wie Einweg- und auch Mehrweg-Instrumente einer Wiederverwertung zugeführt werden können, und zwar ohne dass diese Gegenstände zuvor auszusortieren sind. Durch Verwendung der flexiblen Behältnisse, insbesondere am Ort des Anfalls benutzter und/oder kontaminierter metallischer medizinischer Geräte, kombiniert mit dem Einsatz eines stabilen bzw. starren Metallgebindes, das einem Pressschritt unterworfen wird, gelangt man nicht nur dahin, medizinische Einweg- und Mehrweginstrumente einer materialbezogenen Wiederverwertung zuzuführen. Vielmehr stellt das erfindungsgemäße Verfahren insbesondere auch eine sichere Handhabung benutzter und/oder kontaminierter medizinischer Geräte sowohl beim Klinikpersonal wie auch bei Mitarbeitern von Transport- und Recyclingunternehmen sicher.

## Patentansprüche

1. Verfahren zur, insbesondere Ressourcen schonenden, Behandlung, insbesondere zur recycelnden Entsorgung, von, insbesondere kontaminierten, metallischen medizinischen Geräten, umfassend die Schritte:
a) Zurverfügungstellung mindestens eines mindestens abschnittsweise flexiblen, verschließbaren Sammelbehältnisses für benutzte und/oder kontaminierte metallische medizinische Geräte und Zurverfügungstellung mindestens eines verschließbaren Metallgebindes eingerichtet und ausgelegt zur Aufnahme von mindestens zwei, insbesondere einer Vielzahl an verschlossenen mindestens abschnittsweise flexiblen Sammelbehältnissen, enthaltend benutzte und/oder kontaminierte metallische medizinische Geräte,
b) Sammeln von benutzten und/oder kontaminierten metallischen medizinischen Geräten in dem mindestens einen mindestens abschnittsweise flexiblen, verschließbaren Sammelbehältnis,
c) gegebenenfalls Verschließen, insbesondere irreversibles Verschließen, des mit benutzten und/oder kontaminierten metallischen medizinischen Geräten befüllten mindestens einen mindestens abschnittsweise flexiblen Sammelbehältnisses,
d) Überführen des mindestens einen, insbesondere verschlossenen, mindestens abschnittsweise flexiblen Sammelbehältnisses in das verschließbare, insbesondere starre, Metallgebinde,
e) Verschließen des mit dem mindestens einen verschlossenen mindestens abschnittsweise flexiblen Sammelbehältnis befüllten Metallgebindes,
f) Unterwerfen des mindestens einen verschlossenen Metallgebindes mindestens einem Pressschritt zur Volumenreduktion unter Erhalt eines verpressten Gebildes und
g) Aufschmelzen des Metalls des verpressten Gebildes enthaltend das Metall des Metallgebindes und das Metall der metallischen medizinischen Geräte und Rückgewinnung der aufgeschmolzenen Metalle.

2. Verfahren nach Anspruch 1, ferner umfassend
h) die Herstellung von metallischen medizinischen Geräten und/oder von metallischen Gebinden aus den in Schritt g) rückgewonnenen Metallen.

3. Verfahren nach Anspruch 1 oder 2, **dadurch gekennzeichnet, dass** das mindestens abschnittsweise flexible, verschließbare Sammelbehältnis im Wesentlichen feuchtigkeitsbeständig, dicht, insbesondere flüssigkeitsdicht, perforationsresistent, insbesondere stichfest, reißfest und/oder dauerhaft verschließbar ist.

4. Verfahren nach einem der vorangehenden Ansprüche, **dadurch gekennzeichnet, dass** das Verschließen des Metallgebindes in Schritt e) den Schritt des Verplombens umfasst.

5. Verfahren nach einem der vorangehenden Ansprüche, **dadurch gekennzeichnet, dass** in Schritt f) jeweils ein einzelnes verschlossenes Metallgebinde oder eine Vielzahl verschlossener Metallgebinde dem Pressschritt, insbesondere unter Ausbildung des verpressten, insbesondere im Wesentlichen zylinderförmigen, Gebildes unterworfen wird.

6. Verfahren nach einem der vorangehenden Ansprüche, **dadurch gekennzeichnet, dass** eine Vielzahl verschlossener, befüllter Metallgebinde gemäß Schritt e) zu einem ersten Lagerort, insbesondere umfassend eine Metallpresse, transportiert werden und/oder dass eine Vielzahl verpresster Gebilde gemäß Schritt f) zu einem zweiten Lagerort, insbesondere umfassend eine Metallschmelze, transportiert werden.

7. Verfahren nach einem der vorangehenden Ansprüche, **dadurch gekennzeichnet, dass** das Metallgebinde ein Stahlgebinde ist oder umfasst und/oder dass die flexiblen Wandungsmaterialien des mindestens abschnittsweise flexiblen Sammelbehältnisses ausgewählt sind aus der Gruppe bestehend aus Polyolefin-Faservlieslagen, Laminatsystemen, enthaltend mindestens eine Papierlage und/oder mindestens eine feuchtigkeitsbeständig beschichtete weitere Papierlage und/oder mindestens eine Kunststofffolienlage, insbesondere enthaltend mindestens eine Papierlage und mindestens eine Kunststofffolienlage und/oder mindestens eine feuchtigkeitsbeständig beschichtete weitere Papierlage, ein- oder beidseitig in eine Kunststofflage eingebetteten Metallgeweben, Metallgewirken oder Metallvliesen und Lagen aus Polyvinylbutyrat, insbesondere recyceltem Polyvinylbutyrat sowie deren beliebigen Mischungen.

8. Verfahren nach einem der vorangehenden Ansprüche, **dadurch gekennzeichnet, dass** die metallischen medizinischen Geräte metallische Einweg-Instrumente oder, insbesondere chirurgische, metallische Mehrweg-Instrumente darstellen oder umfassen.

9. Verfahren nach einem der vorangehenden Ansprüche, **dadurch gekennzeichnet, dass** die mindestens abschnittsweise flexiblen, verschließbaren Sammelbehältnisse für benutzte und/oder kontaminierte metallische medizinische Geräte in Kliniken, medizinischen Ambulanzen und/oder Arztpraxen zur Verfügung gestellt werden.

10. Verfahren nach einem der vorangehenden Ansprüche, **dadurch gekennzeichnet, dass** die mindestens abschnittsweise flexiblen, verschließbaren Sammelbehältnisse eingerichtet und ausgelegt sind, um an einem Stationswagen temporär aufgehängt zu werden.

11. Verfahren nach einem der vorangehenden Ansprüche, **dadurch gekennzeichnet, dass** die mindestens abschnittsweise flexiblen, verschließbaren Sammelbehältnisse eingerichtet und ausgelegt sind, um benutzte und/oder kontaminierte metallische medizinische Geräte mit einem Gesamtgewicht im Bereich von 0,5 bis 5,0 kg, insbesondere im Bereich von 1,0 bis 3,0 kg, aufzunehmen, und/oder dass das verschließbare Metallgebinde ein Aufnahmevolumen im Bereich von 50 bis 250 1, insbesondere im Bereich von 100 bis 200 1, aufweist.

12. Verfahren nach einem der vorangehenden Ansprüche, **dadurch gekennzeichnet, dass** die metallischen medizinischen Geräte Chrom und/oder Nickel enthalten, insbesondere mit Chrom und/oder Nickel legierte Edelstähle darstellen oder umfassen.

13. Verfahren nach einem der vorangehenden Ansprüche, **dadurch gekennzeichnet, dass** die mindestens abschnittsweise flexiblen verschließbaren Sammelbehältnisse, insbesondere ausschließlich, nicht-infektiös kontaminierte metallische Abfälle, insbesondere gemäß Abfallschlüssel AS 180104 nach Merkblatt Nr. 18 der Richtlinie über die ordnungsgemäße Entsorgung von Abfällen aus Einrichtungen des Gesundheitsdienstes gemäß Bundesgesundhbl. 2002, 51: 234-241, der Länder-Arbeitsgemeinschaft Abfall (LAGA) enthalten.

14. Sammelbehältnis für metallische medizinische Geräte, umfassend eine Behältnisvorderwand, eine Behältnisrückwand,
ein unteres Behältnisende, umfassend einen Behältnisboden, und
einen dem Behältnisboden gegenüberliegendes verschlossenes oberes Behältnisende, wobei näher beabstandet zum verschlossenen oberen Behältnisende als zum Behältnisboden in einem Abschnitt der Behältnisvorderwand oder der Behältnisrückwand eine Behältnisöffnung vorliegt, die sich in Richtung von einer ersten Behältnisseitenwand zu einer, der ersten Behältnisseitenwand gegenüberliegenden zweiten Behältnisseitenwand erstreckt,
ferner umfassend mindestens ein Verschlusselement an oder auf der Behältniswand, welche die Behältnisöffnung enthält, diesseits der Behältnisöffnung in Bezug auf das obere Behältnisende, ausgelegt und eingerichtet, um die Behältnisöffnung zu verschließen, und mindestens eine Haltevorrichtung, ausgelegt und eingerichtet zum temporären Befestigen des Sammelbehältnisses, so dass bei gattungsgemäßem Gebrauch die Behältnisöffnung unterhalb des oberen Behältnisendes vorliegt,
wobei Behältnisvorderwand und Behältnisrückwand am unteren Behältnisende einstückig ineinander übergehen oder
wobei Behältnisvorderwand und Behältnisrückwand am unteren Behältnisende in einem sich von der ersten Behältnisseitenwand bis zur zweiten Behältnisseitenwand erstreckenden überlappenden ersten Abschnitt miteinander verbunden vorliegen und wobei der überlappende verbundene erste Abschnitt vollständig oder teilweise umgeschlagen auf und verbunden mit der Behältnisvorderwand oder die Behältnisrückwand vorliegt, und
wobei Behältnisvorderwand und Behältnisrückwand am verschlossenen oberen Behältnisende in einem sich von der ersten Behältnisseitenwand bis zur zweiten Behältnisseitenwand erstreckenden überlappenden zweiten Abschnitt zumindest teilweise miteinander verbunden vorliegen und wobei der überlappende zumindest teilweise verbundene zweite Abschnitt vollständig oder teilweise umgeschlagen auf und verbunden mit der Behältnisvorderwand oder die Behältnisrückwand vorliegt oder wobei ein am oberen Behältnisende über die Behältnisrückwand hinausragender Abschnitt der Behältnisvorderwand unter Ausbildung des verschlossenen oberen Endes umgeschlagen auf und verbunden mit der Behältnisrückwand vorliegt oder wobei ein am oberen Behältnisende über die Behältnisvorderwand hinausragender Abschnitt der Behältnisrückwand unter Ausbildung des verschlossenen oberen Endes umgeschlagen auf und verbunden mit der Behältnisvorderwand vorliegt.

15. Sammelbehältnis nach Anspruch 14, **dadurch gekennzeichnet, dass** die erste Behältnisseitenwand eine flexible Behältnisseitenwand darstellt, umfassend mindestens eine Seitenfalte, oder dass die zweite Behältnisseitenwand eine flexible Behältnisseitenwand darstellt, umfassend mindestens eine Seitenfalte.

16. Sammelbehältnis nach Anspruch 14 oder 15, **dadurch gekennzeichnet, dass** dieses ein Sammelbehältnis für metallische chirurgische Einweginstrumente ist.

## Claims

1. A method for the, in particular resource-saving, treatment, in particular for disposal for recycling, of, in particular contaminated, metallic medical devices, comprising the following steps:
a) Providing at least one lockable collecting container, that is flexible at least in sections, for used and/or contaminated metallic medical devices and the provision of at least one lockable metal receptacle, installed and designed to hold at least two, in particular a plurality of locked collecting containers that are flexible at least in sections, containing used and/or contaminated metal medical devices,
b) Collecting used and/or contaminated metallic medical devices in the at least one lockable collecting container that is flexible at least in sections,
c) If appropriate, locking, in particular irreversible locking, the at least one collecting container that is filled with used and/or contaminated metallic medical devices which is flexible at least in sections,
d) Transferring the at least one, in particular locked, collection container that is flexible at least in sections, into the lockable, in particular rigid, metal receptacle,
e) Locking the metal receptacle that is filled with the at least one locked collection container that is flexible at least in sections,
f) Subjecting the at least one locked metal receptacle to at least one compression step for volume reduction, while retaining a compressed structure, and
g) Melting the metal of the compressed structure containing the metal of the metal receptacle and the metal of the metallic medical devices and recovering the melted metals.

2. The method according to Claim 1, further comprising
h) Producing metallic medical devices and/or metallic receptacles from the metals recovered in step g).

3. The method according to Claim 1 or 2, **characterized in that** the lockable collecting container that is flexible at least in sections is essentially moisture-resistant, in particular fluid-tight, perforation-resistant, in particular cut-proof, tear-proof and/or permanently lockable.

4. The method according to any one of the preceding claims, **characterized in that** the locking of the metal receptacle in step e) comprises the step of sealing.

5. The method according to any one of the preceding claims, **characterized in that** in step f), in each case one single locked metal receptacle or a plurality of locked metal receptacles is subjected to the compression step, in particular to form the compressed, in particular essentially cylindrical, structure.

6. The method according to any one of the preceding claims, **characterized in that** a plurality of locked, filled metal receptacles according to step e) are transported to a first storage site, in particular comprising a metal compressor, and/or that a plurality of compressed structures according to step f) are transported to a storage site, in particular comprising a metal smelting facility.

7. The method according to any one of the preceding claims, **characterized in that** the metal receptacle is or comprises a steel receptacle and/or that the flexible wall materials of the collecting container that is flexible at least in sections are selected from the group consisting of polyolefin fibre fleece layers, laminate systems, containing at least one paper layer and/or at least one moisture-resistant coated further paper layer and/or at least one plastic foil layer, in particular containing at least one paper layer and at least one plastic foil layer and/or at least one moisture-resistant coated further paper layer, metal fabrics embedded on one or both sides in a plastic layer, metal knitted fabrics or metal fleeces and layers of polyvinyl butyrate, in particular recycled polyvinyl butyrate and any mixtures of these.

8. The method according to any one of the preceding claims, **characterized in that** the metallic medical devices are or comprise disposable metallic instruments, or in particular, surgical, metallic reusable instruments.

9. The method according to any one of the preceding claims, **characterized in that** the lockable collecting containers that are flexible at least in sections for used and/or contaminated metallic medical devices are made available for use in clinics, medical outpatient clinics and/or doctors' practices.

10. The method according to any one of the preceding claims, **characterized in that** the lockable collecting containers that are flexible at least in sections are installed and designed to be temporarily suspended on a ward trolley.

11. The method according to any one of the preceding claims, **characterized in that** the lockable collecting containers that are flexible at least in sections are installed and designed to hold used and/or contaminated metallic medical devices with a total weight ranging from 0.5 to 5.0 kg, in particular in the range of 1.0 to 3.0 kg, and/or that the lockable metallic receptacle has a retention volume ranging from 50 to 250 1, in particular ranging from 100 to 200 1.

12. The method according to any one of the preceding claims, **characterized in that** the metallic medical devices contain chrome and/or nickel, in particular are or comprise chrome and/or nickel-alloyed stainless steels.

13. The method according to any one of the preceding claims, **characterized in that** the lockable collecting containers that are flexible at least in sections in particular contain exclusively non-infectiously contaminated metallic waste, in particular in accordance with the waste code AS 180104 as defined in data sheet no. 18 of the guideline on the correct disposal of waste from healthcare institutions in compliance with the Federal Health Bulletin 2002, 51: 234-241 of the Federal States Working Group for Waste (Länder-Arbeitsgemeinschaft Abfall; LAGA).

14. A collecting container for metallic medical devices, comprising a container front wall, a container rear wall, a lower container end, comprising a container floor and a locked upper container end opposite the container floor, whereby at a closer distance from the locked upper container end than from the container floor, in a section of the container front wall or the container rear wall, a container opening is provided that extends in the direction of a first container wall to a second container wall that lies opposite the first container wall,
further comprising at least one locking element at or on the container wall which contains the container opening, this side of the container opening in relation to the upper container end, designed and installed to lock the container opening, and at least one holding device, designed and installed for the temporary affixation of the collecting container, so that with the generic use, the container opening lies below the upper container end,
whereby the container front wall and the container rear wall merge into each other on the lower container end to form a single part,
or whereby the container front wall and the container rear wall are interconnected on the lower container end in an overlapping first section that extends from the first container side wall to the second container side wall, and whereby the overlapping connected first section is entirely or partially turned over on and connected with the container front wall or the container rear wall, and
whereby the container front wall and the container rear wall on the locked upper container end are at least partially interconnected in an overlapping section that extends from the first container side wall to the second container side wall, and whereby the overlapping at least partially connected second section is entirely or partially turned over on and connected to the container front wall or the container rear wall, or whereby a section of the container front wall that protrudes on the upper container end over the container rear wall is turned over on and connected to the container rear wall to form the locked upper end, or whereby a section of the container rear wall that protrudes over the container front wall is turned over on and connected to the container rear wall to form the locked upper end.

15. The collecting container according to Claim 14, **characterized in that** the first container side wall is a flexible container side wall, comprising at least one side fold, or that the second container side wall is a flexible container side wall comprising at least one side fold.

16. The collecting container according to Claims 14 or 15, **characterized in that** this is a collecting container for metallic surgical disposable instruments.

## Revendications

1. Procédé de traitement, en particulier économique en ressources, en particulier de dépollution par valorisation, en particulier des appareils médicaux métalliques contaminés, comprenant les étapes suivantes :
a) la mise à disposition d'au moins un récipient collecteur, au moins par sections souple, pouvant être fermé, pour appareils médicaux métalliques usés et/ou contaminés et la mise à disposition d'au moins un contenant métallique, pouvant être fermé, disposé et conçu pour recevoir au moins deux, en particulier une pluralité de récipients collecteurs, au moins par sections souples, pouvant être fermés, contenant les appareils médicaux métalliques usés et/ou contaminés ;
b) la collecte des appareils médicaux métalliques usés et/ou contaminés dans l'au moins un récipient collecteur, au moins par sections souple, pouvant être fermé ;
c) la fermeture, éventuellement, en particulier la fermeture de manière irréversible, du récipient collecteur, au moins par sections souple, rempli des appareils médicaux métalliques usés et/ou contaminés ;
d) le transfert de l'au moins un récipient collecteur, au moins par sections souple, en particulier fermé, vers le récipient métallique, pouvant être fermé, en particulier rigide ;
e) la fermeture du récipient métallique rempli de l'au moins un récipient collecteur fermé, au moins par sections souple ;
f) la soumission de l'au moins un récipient métallique fermé à au moins une étape de compression destinée à la réduction du volume pour obtenir une structure comprimée ; et
g) la fusion du métal de la structure comprimée contenant le métal du récipient métallique et le métal des appareils médicaux métalliques et la récupération des métaux fondus.

2. Procédé selon la revendication 1, comprenant en outre
h) la fabrication d'appareils médicaux métalliques et/ou de récipients métalliques à partir des métaux récupérés dans l'étape g).

3. Procédé selon la revendication 1 ou 2, **caractérisé en ce que**
le récipient collecteur, au moins par sections souple, pouvant être fermé, est sensiblement résistant à l'humidité, étanche, en particulier étanche aux liquides,
résistant à la perforation, en particulier résistant à la coupure, à la déchirure et/ou pouvant être fermé de manière permanente.

4. Procédé selon l'une quelconque des revendications précédentes, **caractérisé en ce que** la fermeture du récipient métallique comprend dans l'étape e) une étape de mise sous plomb.

5. Procédé selon l'une quelconque des revendications précédentes, **caractérisé en ce que** dans l'étape 1) un différend récipient métallique fermé respectif ou une pluralité de récipients métalliques fermés sont soumis à l'étape de compression, en particulier pour obtenir une structure comprimée, en particulier essentiellement cylindrique.

6. Procédé selon l'une quelconque des revendications précédentes, **caractérisé en ce qu'**une pluralité de récipients métalliques fermés remplis, selon l'étape e), sont transportés vers un premier lieu de stockage, en particulier comprenant une presse métallique et/ou une pluralité de structures comprimées, selon l'étape f), sont transportées vers un deuxième lieu de stockage, en particulier comprenant un métal fondu.

7. Procédé selon l'une quelconque des revendications précédentes, **caractérisé en ce que** le récipient métallique est ou comprend un récipient en acier et/ou que des matériaux de la paroi souple du récipient collecteur, au moins par sections souple, sont choisis dans le groupe constitué par des couches de non-tissés en polyoléfine, des systèmes stratifiés comprenant au moins une couche de papier et/ou au moins une couche de papier revêtue résistant à l'humidité et/ou au moins une couche de film plastique, en particulier comprenant au moins une couche de papier et au moins une couche de film plastique et/ou au moins une couche de papier revêtue résistant à l'humidité, des tissus métalliques incorporés d'une ou de deux côtés dans une couche de matière plastique, des mailles métalliques ou des non-tissés métalliques et des couches de polyvinylbutyrate, en particulier du polyvinylbutyrate valorisé ainsi que leurs mélanges souhaités.

8. Procédé selon l'une quelconque des revendications précédentes, **caractérisé en ce que** les appareils médicaux métalliques représentent ou comprennent des instruments jetables métalliques ou, en particulier des instruments chirurgicaux réutilisables métalliques.

9. Procédé selon l'une quelconque des revendications précédentes, **caractérisé en ce que** les récipients collecteurs, au moins par sections souples, pouvant être fermés, pour appareils médicaux métalliques usés et/ou contaminés, sont mis à disposition dans des cliniques, des ambulances médicales et/ou des cabinets médicaux.

10. Procédé selon l'une quelconque des revendications précédentes, **caractérisé en ce que** les récipients collecteurs, au moins par sections souples, pouvant être fermés, sont disposés et conçus pour être temporairement suspendus à un chariot de service médical.

11. Procédé selon l'une quelconque des revendications précédentes, **caractérisé en ce que** les récipients collecteurs, au moins par sections souples, pouvant être fermés, sont disposés et conçus pour recevoir les appareils médicaux métalliques usés et/ou contaminés d'un poids total compris dans la plage de 0,5 et 5,0 kg, en particulier dans la plage de 1,0 et 3,0 kg et/ou que le récipient métallique, pouvant être fermé, comporte un volume de réception dans la plage de 50 à 250 1, en particulier dans la plage de 1 00 à 2001.

12. Procédé selon l'une quelconque des revendications précédentes, **caractérisé en ce que** les appareils médicaux métalliques représentent ou comprennent des aciers inoxydables contenant du chrome et/ou du nickel, en particulier alliés au chrome et/ou au nickel.

13. Procédé selon l'une quelconque des revendications précédentes, **caractérisé en ce que** les récipients collecteurs, au moins par sections souples, pouvant être fermés, contiennent, en particulier exclusivement, des déchets métalliques contaminés non infectieux, en particulier conformément à la clé de déchet AS 180104, selon la feuille d'instruction n° 18 de la directive sur l'élimination appropriée des déchets des établissements de santé, conformément au journal du ministère fédéral de la santé 2002, 51 : 234-241 de la Länder Arbeitsgemeinschaft Abfall (LAGA).

14. Récipient collecteur pour appareils médicaux métalliques, comprenant :
une paroi avant du récipient, une paroi arrière du récipient,
une extrémité inférieure du récipient, comprenant un fond de récipient et
une extrémité supérieure du récipient fermée, opposée au fond de récipient,
une ouverture de récipient, se trouvant, espacée, plus près de l'extrémité supérieure du récipient fermée que du fond de récipient, dans une section de la paroi avant du récipient ou de la paroi arrière du récipient, laquelle s'étend dans la direction d'une première paroi de récipient vers une deuxième paroi latérale du récipient, opposée à la première paroi latérale du récipient,
comprenant en outre au moins un élément de fermeture à ou sur la paroi de récipient,
laquelle comprend l'ouverture de récipient, d'un côté de l'ouverture de récipient relatif à l'extrémité supérieur du récipient, disposé et conçu pour fermer l'ouverture de récipient et au moins un dispositif de maintien, disposé et conçu pour fixer temporairement le récipient collecteur de telle sorte que l'ouverture de récipient se trouve, lors de l'utilisation générique, sous l'extrémité supérieure du récipient,
ladite paroi avant du récipient et ladite paroi arrière du récipient, à l'extrémité inférieure du récipient, se confondant mutuellement de manière à former une seule pièce ou ladite paroi avant du récipient et ladite paroi arrière du récipient, à l'extrémité inférieure du récipient, se trouvant raccordées l'une à l'autre, dans une première section se chevauchant, s'étendant de la première paroi latérale du récipient à la deuxième paroi latérale du récipient et ladite première section se chevauchant raccordée se trouvant totalement ou partiellement rabattue sur et raccordée à la paroi avant du récipient ou à la paroi arrière du récipient, et
ladite paroi avant du récipient et ladite paroi arrière du récipient se trouvant, à l'extrémité supérieure du récipient fermée, au moins partiellement raccordées l'une à l'autre, dans une deuxième section se chevauchant, s'étendant de la première paroi latérale du récipient vers la deuxième paroi latérale du récipient et la deuxième section se chevauchant, au moins partiellement raccordée, se trouvant totalement ou partiellement rabattue et raccordée à la paroi avant du récipient ou à la paroi arrière du récipient ou une section de la paroi avant du récipient, faisant saillie à l'extrémité supérieur du récipient sur la paroi arrière du récipient, formant l'extrémité supérieure fermée, se trouvant rabattue et raccordée à la paroi arrière du récipient ou une section de la paroi arrière du récipient, faisant saillie à l'extrémité supérieur du récipient sur la paroi avant du récipient, formant l'extrémité supérieure fermée, se trouvant rabattue et raccordée à la paroi avant du récipient.

15. Récipient collecteur selon la revendication 14, **caractérisé en ce que** la première paroi latérale du récipient représente une paroi latérale du récipient souple, comprenant au moins un pli latéral ou que la deuxième paroi latérale du récipient représente une paroi latérale du récipient souple, comprenant au moins un pli latéral.

16. Récipient collecteur selon la revendication 14 ou 15, **caractérisé en ce que** ledit récipient collecteur est un récipient collecteur pour instruments jetables chirurgicaux métalliques.
